# EUROPEAN PATENT APPLICATION

(11) **EP 4 759 223 A1**
(43) Date of publication of application: **17.06.2026**
(21) Application number: 24220307.3
(22) Date of filing: 16.12.2024
(51) Int. Cl.: A61B 5/00, A61B 5/145

(54) **CONTINUOUS GLUCOSE MONITORING (CGM) DEVICE**

(71) Applicant: Primewise Teknoloji A.S., 34218 Istanbul (TR)
(72) Inventor: CALAYIR, Mehmet Halit, 34218 Istanbul (TR); ÖZTÜRK, TARIK, 34218 Istanbul (TR); ÖZTÜRK, ÖMER, 34218 Istanbul (TR)
(74) Representative: Bakirci, Utkan Bahri

(57) **Abstract**

This invention relates to medical devices, particularly a continuous glucose monitoring (CGM) device designed for the diagnosis and monitoring of metabolic diseases such as diabetes. The invention offers an innovative monitoring solution for both type 1 diabetes, which arises from insufficient insulin production, and the more common type 2 diabetes, which is associated with insulin resistance. By enabling continuous blood sugar monitoring, the invention provides a user-friendly, eco-friendly, and cost-effective solution for diabetes management. The device allows real-time monitoring of glucose levels and delivers visual, auditory, and vibrational alerts to the user. As a result, it establishes a safer, more practical, and continuous health management system for users.

## Description

### Technical Field

This invention relates to the field of medicine, specifically to a medical device for the diagnosis and monitoring of metabolic diseases such as diabetes. The medical device according to the present invention concerns a sensor applicator assembly for the continuous measurement of sugar levels in the human body. Diabetes is a chronic metabolic disease characterized by prolonged high blood sugar levels, which over time can lead to serious complications affecting the heart, blood vessels, eyes, kidneys, and nerves. The present invention specifically offers solutions to challenges associated with type 2 diabetes, which occurs due to insulin resistance or insufficient insulin production. Type 2 diabetes is widespread globally, and its diagnosis is often delayed due to mild symptoms. Furthermore, present invention is also intended for type 1 diabetes, which arises from insufficient or no insulin production by the pancreas. Continuous and appropriate treatments are critical for managing type 1 diabetes. The present invention provides an innovative solution for continuously monitoring sugar levels in the human body, offering an environmentally friendly, user-friendly, and cost-effective approach. More specifically, by applying the sensor module to the body through an applicator, the precise and safe attachment of the sensor module to the body is facilitated. It is possible to attach the sensor module to the body simply by activating the applicator, and the reusability of the applicator contributes to an environmentally friendly solution.

### Background

Diabetes develops as a result of insufficient or relatively inadequate insulin production by the pancreas due to various factors such as obesity, stress, poor dietary habits, and hereditary factors, leading to an inability to regulate blood sugar levels. Throughout history, diabetes has been defined and treated in various ways. The Ebers Papyrus, written in Ancient Egypt around 1500 BCE, refers to symptoms of diabetes such as frequent urination and excessive thirst. The ancient Greek physician Aretaeus of Cappadocia was the first to name the disease "diabetes" and associated it with sugar metabolism after noticing the sweetness of urine. During the Middle Ages, Ibn Sina (Avicenna) provided a detailed description of diabetes symptoms, including sugar accumulation in urine and slow wound healing. However, no effective treatment was developed during this period.

In the 19th century, research revealed the relationship between diabetes and pancreatic dysfunction. In 1869, Paul Langerhans discovered specific cell clusters in the pancreas, and in 1889, Joseph von Mering and Oskar Minkowski demonstrated that removing the pancreas caused diabetes. Until the 20th century, diabetes remained a difficult-to-manage and fatal disease.

By the early 20th century, the fundamental mechanisms of diabetes were partially understood. Particularly, the role of the pancreas in diabetes had been proven through experiments conducted in the late 19th century. However, no specific treatment method had been identified. To address this critical issue, Canadian surgeon Frederick Banting and his assistant Charles Best succeeded in isolating the hormone insulin in 1921. This discovery laid the foundation for modern diabetes treatment. Initially, Banting based his work on the hypothesis that diabetes resulted from a deficiency in a hormone secreted by the pancreatic islets of Langerhans. This theory built upon research by scientists investigating the relationship between the pancreas's exocrine and endocrine functions at the time. Banting and Best attempted to isolate the insulin hormone by blocking the pancreas's exocrine functions. In their experiments, they tied off the pancreatic ducts of dogs, deactivating the pancreas's digestive enzymes. Subsequently, they extracted material from the pancreas tissue and injected it into diabetic dogs. They observed that this extract reduced the dogs' blood sugar levels and alleviated their symptoms. This was the first concrete evidence of insulin's blood sugar-regulating effect. To support Banting and Best's work, biochemist James Collip joined the team and refined the insulin extract, making it pure enough for safe use in humans. The team also conducted their experiments in John Macleod's laboratory to commercialize the discovery.

In 1922, a 14-year-old diabetic boy named Leonard Thompson became the first person to receive insulin therapy. The initial insulin injection administered to Leonard was not sufficiently effective due to purity issues. However, the second injection, using Collip's purified insulin extract, successfully lowered Leonard's blood sugar levels and significantly improved his health condition. For their discovery of insulin, Frederick Banting and John Macleod were awarded the Nobel Prize in Physiology or Medicine in 1923. Banting shared half of the prize with his assistant, Charles Best. This breakthrough transformed diabetes from a fatal disease into a manageable chronic condition. From this point forward, the primary challenge shifted to regularly monitoring blood sugar levels to determine the necessity of insulin and to optimally adjust its timing and dosage when required.

In the 20th century, the industrial production of insulin, the development of glucose monitoring devices, and the use of genetically engineered human insulin significantly facilitated diabetes management. Early diagnosis of diabetes is critical for reducing its long-term complications. However, due to the nature of symptoms in type 1 and type 2 diabetes, diagnosis is often challenging. In type 1 diabetes, symptoms appear suddenly and include frequent urination, excessive thirst, constant hunger, weight loss, visual disturbances, and fatigue. While these symptoms generally allow for rapid diagnosis, their sudden onset can make early-stage diagnosis difficult. In type 2 diabetes, symptoms develop more gradually and are milder. Although similar symptoms to type 1 diabetes may be observed, the less pronounced nature of these symptoms often delays diagnosis, and the disease is frequently identified only after complications arise.

In the 21st century, Continuous Glucose Monitoring (CGM) devices and artificial pancreas systems have introduced innovative solutions in diabetes management. However, current technologies have several significant limitations:
The accuracy of these devices can be affected by external factors such as body movement and temperature. Additionally, energy consumption and the need for regular calibration pose challenges for users. Furthermore, both disposable measurement devices and the single-use components of continuous-use devices are known to have negative environmental impacts. At the same time, disposable components also create substantial cost-related challenges.

As mentioned above, existing methods for measuring blood glucose levels often have limitations. While traditional blood glucose monitoring devices require obtaining a blood sample through finger pricking, Continuous Glucose Monitoring (CGM) systems provide a more modern alternative. However, the high cost of CGM systems underscores the need for more accessible and user-friendly solutions. There are two common and medically accepted methods for measuring blood glucose levels:
In the early development of glucose measurement technologies, blood glucose levels were measured using invasive methods. The first glucose monitoring devices, developed in the 1970s, operated by pricking the finger to obtain a drop of blood, which was then placed on a specialized test strip to determine glucose concentration. However, this method has limitations due to its fully invasive nature, the frequent manual intervention required, and its provision of blood glucose level information only at a single point in time. While these devices revolutionized diabetes management, their usage was inconvenient, and users had to endure 15-20 blood draws daily, making the process uncomfortable and labor-intensive.

An example of such measurement devices is described in patent document US4787398A, which explains a blood glucose measurement device and method. Specifically, it details a blood glucose monitoring system in which a blood sample obtained through finger pricking is applied to a test strip, and the glucose concentration is determined using electrochemical methods. The single-use nature of the reactive-lancet unit results in a continuous need for consumables, making it a costly solution for users. Additionally, the manual intervention required during the measurement process increases the likelihood of user errors and makes it more cumbersome compared to automated systems. Calibration requirements, involving both the device's electronic components and chemical reagents, are time-consuming. The device's limited data storage capacity can lead to overwriting older measurements. Lastly, the complex structure of the spring mechanism and optical measurement units makes maintenance and repair challenging, negatively impacting the device's longevity.

CGM systems utilize a sensor equipped with a subcutaneous probe to continuously monitor blood glucose levels throughout the day. These systems offer significant advantages by tracking glucose level trends in real-time and alerting users to dangerous levels before they occur. However, CGM systems have their drawbacks, including high costs, challenges in securely implanting the probe under the skin, and the necessity of discarding single-use components, which contributes to waste and increases expenses.

An example of such measurement devices is patent document US2023301556 A1, which describes a CGM (Continuous Glucose Monitoring) system and method that continuously monitors glucose levels in interstitial fluid using a subcutaneous sensor probe and transmits the data to a receiver device. One significant drawback of the CGM system described in this invention is that the applicator used to insert the sensor under the skin is single-use. The requirement to use a new applicator for each measurement results in additional costs for the user and contributes to environmental waste. The single-use nature of the applicator negatively impacts the system's sustainability and cost-effectiveness. These disadvantages represent critical limitations, increasing the long-term operational costs and environmental impact of the device.

Therefore, the need for an innovative and environmentally friendly system capable of continuous glucose monitoring, enhancing user comfort, and providing energy efficiency remains. In this context, the present invention has developed a continuous glucose monitoring device aimed at addressing these shortcomings and improving the quality of life for diabetes patients.

### Detailed Explanation Of The Invention

The present invention relates to a continuous glucose monitoring (CGM) sensor system developed to continuously and accurately monitor blood sugar levels.

The aim of the invention is to address the shortcomings of existing CGM technologies and to provide a more accessible, cost-effective, environmentally friendly, and user-friendly alternative for diabetes management.

The invention subject to the application relates to a continuous glucose monitoring (CGM) system capable of measuring glucose levels from subcutaneous interstitial fluid. The device collects data from the interstitial fluid under the skin instead of blood, providing an analysis of glucose levels. This method not only offers a more comfortable experience for users but is also based on innovative technology that ensures continuous and accurate measurements. The invention has been designed to be compatible with various types of insulin pumps and pens.

Glucose measurement from interstitial fluid presents a significant advantage over traditional blood-based measurement methods. Blood glucose levels are highly sensitive to instantaneous changes, which can sometimes lead to misleading results. However, measurements taken from interstitial fluid reflect glucose level changes in a more balanced manner. This reduces the impact of rapid fluctuations in glucose levels, providing a more reliable data set and delivering more accurate information for long-term glucose management.

The continuous glucose monitoring device related to the present invention, in its most basic form, comprises a sensor (1) module containing a glucose sensor probe placed under the skin and a mechanical applicator (2) system that ensures the secure and accurate placement of the sensor module. The applicator (2) has an ergonomic structure that prioritizes user comfort, enabling the sensor (1) module to be positioned in a stable, precise, and effective manner. This aims to make the subcutaneous placement process both easy and reliable.

The sensor (1) module developed for the device subject to the invention includes a probe with a three-layer electrode placed under the skin and an electronic system that analyzes the voltage values from these electrodes. The first layer of the three-layer electrode is referred to as the "reference electrode." This electrode is used to measure the existing voltage level in the body. The measured voltage value is processed through an integrated circuit and a higher voltage is generated, which is then sent to the second electrode layer, known as the "working electrode." The third and final electrode, called the "counter electrode," generates a lower voltage. This creates a voltage difference between the working electrode and the counter electrode.

The surface of the glucose sensor probe is initially coated with the enzyme glucose oxidase to ensure interaction exclusively with glucose. However, high concentrations of glucose and proteins can block this enzyme, potentially reducing its efficiency. To preserve the enzyme's effectiveness, a specialized biocompatible TPU (Thermoplastic Polyurethane) film is applied as the outermost layer. This additional coating significantly extends the lifespan of the probe, contributing to the long-term and reliable operation of the sensor module.

In the preferred embodiments of the invention, biocompatible TPU types that can be used to protect and extend the lifespan of the enzyme-coated surface of the probe are typically thermoplastic polyurethanes with a block copolymer structure. These TPUs are produced using aromatic diisocyanates (e.g., 4,4'-Methylenediphenyl diisocyanate, MDI), polyether or polycarbonate-based polyols (e.g., polycarbonate diols, polytetramethylene ether glycol - PTMEG), and short-chain diols (e.g., 1,4-butanediol). In this structure, MDI-based hard segments provide structural durability and stability, while polycarbonate or polyether-based soft segments enhance resistance to hydrolysis and protein adsorption under prolonged biological contact conditions. As a result, by using a polyether-based TPU composed of MDI/PTMEG/1,4-butanediol or a polycarbonate-based TPU composed of MDI/polycarbonate diol/1,4-butanediol, high biocompatibility, chemical resistance, and mechanical stability can be achieved. This specialized TPU film, forming the outermost layer of the glucose sensor probe, supports the long-term reliable and efficient operation of the sensor module.

In one embodiment of the invention, to obtain a medical-grade, biocompatible TPU, a combination of 4,4'-Methylenediphenyl diisocyanate (MDI) and 1,4-butanediol (1,4-BDO) can be used for the hard segment structure, while the soft segment is preferably derived from a polycarbonate-based polyol (preferably a polycarbonate diol with a molecular weight of approximately 1000-1500 g/mol). A TPU composition prepared with approximately 20-25% MDI, 5-10% BDO, and 65-75% polycarbonate diol by total weight can result in a medium-hard, flexible yet durable film. To control the polymerization reaction, a suitable catalyst (preferably tin- or amine-based) can be added in small amounts (approximately 0.1-0.5%). Additionally, for enhancing long-term stability, medical-grade additives such as antioxidants or UV stabilizers can be included in low concentrations.

The sensor (1) module comprises a transmitter for transmitting the measured blood glucose level. In a preferred embodiment of the invention, the transmitter is configured to be reusable.

Among the other structural and functional elements of the sensor (1) module are a top plastic cover that protects the internal components from external effects, a printed circuit board (PCB) that contains the electronic circuits and processor units, a bottom plastic part, an adhesive band used to secure the sensor (1) module to the skin or the desired area, contact points to ensure electrical connectivity, a connection socket that preserves these contact points and provides a secure connection environment, a junction that fits into the connection socket, a battery that meets the energy needs of the module, and an antenna used for wireless data transmission. These elements contribute to the structural integrity and durability of the sensor (1) module, ensuring reliable and long-lasting operation by enabling data processing and transmission capabilities.

The main body of the applicator (2) is produced with an ergonomic structure to allow easy handling by the user. The body includes a triggering mechanism that activates simply by pressing the applicator (2) during the placement of the sensor (1) module and a slot to hold the sensor (1) securely in place during the placement process. The automatic triggering mechanism (insertion mechanism) ensures that the glucose sensor probe is inserted under the skin quickly and in a controlled manner, minimizing any discomfort to the user during the procedure. The sensor (1) slot ensures that the sensor (1) remains securely fixed during placement.

The device subject to the invention includes a sensor placement structure that uses a hands-free, replaceable guide needle (3), adapted to penetrate the skin rapidly with the assistance of a spring-loaded mechanical push mechanism. This mechanism, which can be reset and activated by pressing the device against the skin, inserts the glucose sensor probe under the skin at a predetermined angle and depth. The guide needle (3) directs the insertion (placement) of the glucose sensor probe beneath the skin and is removed from the system once the placement process is completed. The guide needle (3) is typically made of stainless steel or a biocompatible material. In one embodiment, the guide needle (3) features an internal channel structure into which the electrode is placed. To enhance user safety and prevent unnecessary exposure, a needle retraction mechanism is implemented. This mechanism automatically retracts the guide needle (3) into the applicator body upon completion of the placement process, ensuring user safety. A needle removal button located in the insertion mechanism of the applicator (2) activates the spring that retracts and removes the guide needle (3) hands-free, enabling its safe disposal.

One embodiment of the present invention relates to a continuous body glucose monitoring system in which a sensor (1) (designed to measure glucose in the body at regular intervals) is integrated into an applicator (2) and combined as a single unit. The activation of the applicator (2) allows the sensor module to be secured to the body. In this system, an additional section containing an adhesive band is attached to the underside of the sensor (1) module. When the applicator (2) is activated, this section is pushed outward, enabling the sensor (1) module to be fixed to the body. A glucose sensor probe located within this section is positioned so that its lower tip enters the body when the sensor (1) module is deployed, allowing access to body fluids. Additionally, a detachable guide needle (3) is inserted into the body along with the tip of the glucose sensor probe. Once the sensor module is secured to the skin, the guide needle retracts and exits the body, leaving the glucose sensor probe inside to monitor continuously. This mechanism facilitates the application, placement, and use of the sensor, enabling continuous glucose monitoring to be performed more practically and reliably.

In a preferred embodiment of the invention, the applicator (2) includes a mechanism that enables the sensor (1) module to move linearly from a first position to a second position when the user presses a button or applies pressure to the device. This structure features a push button or a mechanism on the main body that is easily accessible to the user. Initially, the sensor (1) module is attached to the first position of the main body. When the push button is pressed, this connection is released, allowing the sensor (1) module to move linearly outward to the second position. This movement is facilitated by a piston body, which moves forward along with the sensor (1) module, and a spring that applies elastic force to the piston body, triggering the linear motion. Additionally, the system incorporates a needle retraction mechanism that activates once the sensor module reaches the second position. This mechanism moves the needle section upward, retracting the needle from the body. As a result, with a single simple action, the user can apply the sensor (1) and retract the needle, while the sensor component necessary for measurement remains in the body.

In this mechanism, the needle retraction system includes a needle retraction body connected to the piston body. This needle retraction body moves linearly along with the piston body from the first position to the second position and its lower end locks with the upper end of the needle section. Additionally, a needle retraction spring applies an upward elastic force to the needle retraction body. When the needle retraction body reaches the second position, the needle section is detached from the body, and the needle is pulled upward by the force of the needle retraction spring, thereby being removed from the body.

The main body consists of an outer body with a push button on one side that the user can press, and an inner body positioned within the outer body, which guides the linear motion path of the piston body.

The needle retraction body features a flexible hook designed to lock with the piston body in a detachable manner. When the needle retraction body reaches the second position, a needle retraction pressure section may be present to ensure the proper positioning of the hook by pressing it into place for secure locking.

Additionally, a cushioning element can be placed at the upper part of the inner surface of the main body. This cushioning element reduces the impact of the upward elastic motion of the needle retraction body, minimizing the force of the resulting shock.

The needle retraction body includes a needle head connection part that attaches to the upper end of the needle section. On the needle retraction body, there may also be a needle support section that ensures the needle remains stationary relative to the body and secures its position. Additionally, since the needle section can be completely detached from the system after application, the user can easily replace the needle with a new one.

With this invention, the integration of the sensor module with the applicator assembly minimizes the user actions required to place the sensor module on the body, allowing the sensor to be applied with a simple button press. Thus, the sensor module can be easily attached to the body without requiring additional effort from the user.

Additionally, the placement of the battery within the sensor module and the ability of an external transmitter to utilize the power from the sensor module in a preferred embodiment of the invention provide a cost advantage. Since the sensor module is designed as a single-use unit, it offers a precise, safe, and maintenance-free solution.

Furthermore, as the sensor module is secured to the body by activating the applicator, the automatic retraction of the placement needle prevents any harm to the body during the needle removal process. The needle is withdrawn quickly and painlessly, enhancing user comfort.

In another preferred embodiment of the invention, removing the protective cover automatically detaches the protective paper from the adhesive band of the sensor module. This allows the sensor to be applied to the body without requiring any additional steps beyond removing the protective cover.

When it is necessary to replace the sensor module, a new sensor module provided in a separate package is carefully placed into the applicator after its protective layer is removed. The sensor module is then securely affixed to the skin by activating the applicator.

Additionally, while the sensor module is being placed into the applicator, a separate protective cover is attached to the applicator in a removable and reattachable manner to prevent interaction with the external environment. This ensures that after the sensor module is separated from the protective cover, it can be applied to the body by activating the applicator.

Meanwhile, an adhesive band is attached to the surface of the sensor module that will come into contact with the body. The protective paper on the adhesive band can be designed to automatically detach from the band during the process of removing the protective cover from the applicator. For instance, the protective paper may be adhered to the protective cover on one side. Thus, when the user removes the protective cover from the applicator, the protective paper on the adhesive band is also removed along with it. In this scenario, the sensor module is ready to be adhered to the body without requiring any additional step from the user.

The applicator (2) is configured to allow the sensor module to be inserted and secured within it. When the sensor module (1) is pushed into the applicator, it locks into place at a specific position, ensuring stability. Upon activation of the applicator, the sensor module is carefully guided outward and applied to the body. Once the application process is complete, the applicator detaches from the sensor module, leaving the sensor module adhered to the skin via its adhesive band. This ensures that the sensor module is securely affixed to the body while the placement needle is removed for reuse.

In a preferred embodiment of the invention, the device also comprises a needle ejection button, which is preferably located at the top of the applicator. This applicator, which adheres the sensor to the skin using automatic triggering and inserts the glucose sensor probe subcutaneously with a specially housed needle system, automatically retracts the housed needle via a spring mechanism after the process is completed. The retracted needle is hygienically single-use. Finally, by pressing the needle ejection button at the top, the needle is automatically expelled from the applicator and hygienically discarded into a waste container without being touched by the user. According to an embodiment of the invention, the device is characterized in that the insertion mechanism comprises a needle ejection button which activates a spring to allow the guide needle (3) to be withdrawn and discarded untouched after insertion of the sensor (1) has been completed.

One of the key features of the device subject to the invention is the reusability of the applicator (2). The primary factor enabling this is that the guide needle (3) in the applicator (2) can be replaced hands-free, allowing the applicator to be reset for further use.

In one embodiment of the invention, the applicator (2) includes a child lock, eliminating any potential danger posed by the guide needle (3) to children.

In another embodiment, the sensor (1) features a waterproof outer casing to ensure that the device continues to function even when exposed to skin moisture, sweat, or water.

In yet another embodiment, the device includes a vibration module that is activated when data processed by an external device is transmitted to the device through the data transmission system.

In one embodiment of the invention, a cloud-based backup module is included to ensure that, in the event of disconnection from other devices, sensor data is recorded, transmitted to the user and/or all predefined devices, relevant institutions, remotely accessible digital health platforms, and/or triggers the activation of the vibration module.

The applicator (2) features a special mechanism for preparing and stabilizing the skin in the area where the glucose sensor probe of the sensor (1) will be placed. A skin stabilization ring is used to secure the skin surface and ensure the sensor is positioned at the correct angle. This ring prevents the sensor from moving during placement and creates optimal tension on the skin surface, enhancing the accuracy of the application. As a result, the position of the sensor beneath the skin becomes fully controllable.

In one embodiment of the invention, a 25-gauge guide needle (3) is used. The sensor probe, along with the sensor's circuitry, is adhered to the skin with a waterproof housing after being placed into the body via a spring-loaded placement system.

The spring-loaded placement system ensures the precise and controlled insertion of the glucose sensor probe beneath the skin. This system operates through a two-stage mechanism: the first triggering spring pushes the glucose sensor probe into the subcutaneous layer, while the second release spring ensures that the sensor is automatically left in a stable position once the placement process is complete. This spring mechanism optimizes the placement process for both the user and the device.

After the sensor (1) is placed, an adhesive pad and a protective cover are configured to provide protection against external impacts and environmental effects. The adhesive pad ensures that the sensor (1) remains fixed on the skin, preventing it from being affected by external factors. The protective cover, on the other hand, surrounds the outer part of the sensor (1) and serves as a structure that protects against impacts. This design enhances the stability of the sensor (1) by minimizing slippage or positional changes that may result from user movements.

In one configuration of the invention, the device includes an adhesive structure that fixes the device to the user's skin through an adhesive pad located on the part where the sensor (1) module comes into contact with the skin. This adhesive structure comprises two or more layers, with at least one of these layers directly contacting the skin and containing a skin-friendly adhesive material, and at least one other layer made of a flexible and impact-absorbing (vibration-damping) material. In one configuration of the invention, the adhesive material contained in the layer of the adhesive structure directly contacting the skin includes acrylate/methacrylate copolymer blends, silicone-based adhesives, or hydrogel/hydrocolloid-based adhesives, or mixtures of two or more of these. In another configuration of the invention, the material contained in at least one other layer includes a polyurethane (PU) elastomer or foam layer, a silicone gel layer, a thermoplastic elastomer (TPE) or Santoprene-based layer, or mixtures of two or more of these.

Various chemical and formulation-based solutions can be applied for multilayer adhesive structure. In this context, medical-grade adhesives that are typically biocompatible, hypoallergenic, and easy to remove are preferred for the lower layer, which directly contacts the skin. Preferably, acrylic adhesives containing acrylate or methacrylate copolymers, such as 2-ethylhexyl acrylate, n-butyl acrylate, acrylic acid, and low levels of photo- or free-radical initiators, or polydimethylsiloxane (PDMS)-based silicone adhesives, can be used for this purpose. Silicone-based adhesives are known for their high moisture permeability and low risk of irritation during prolonged contact with the skin. Additionally, hydrogel or hydrocolloid-based adhesives, preferably formulations containing carboxymethyl cellulose, poly(acrylic acid), or polyvinylpyrrolidone, can also be used to provide soft and comfortable contact with the skin while minimizing skin irritation. For the upper layer, materials such as polyurethane, preferably based on poly(ether)diol and MDI+1,4-butanediol, silicone gel, or thermoplastic elastomers (e.g., styrene-ethylene/butylene-styrene copolymers) are used to achieve a flexible, vibration-damping structure. This layer enhances the comfort and reliability of the sensor module on the user's skin by offering high impact absorption capacity and mechanical stability. All these adhesive and layer solutions can be optimized in line with criteria such as sterilization (ETO, gamma, e-beam), long-term contact, and biocompatibility (e.g., ISO 10993). Thus, the multilayer adhesive structure, composed of two or more layers, provides both gentle adhesion to the skin and vibration-damping properties.

The device subject to the invention has been developed to make the sensor placement process safe and user-friendly. The applicator not only ensures that the sensor is placed at the correct depth and angle but also allows the procedure to be completed painlessly and quickly. Furthermore, both the sensor and applicator components are designed to ensure long-term use and durability.

In different configurations of the invention, various types of sensors can be used. Detailed information about these sensors is provided herein below.

Electrochemical sensor probes rely on enzymatic reactions to measure glucose in the interstitial fluid beneath the skin. Coated with enzymes such as glucose oxidase or glucose dehydrogenase, these probes convert glucose molecules into electrical signals through a chemical reaction. When placed under the skin, these sensor probes can provide continuous measurements with high accuracy. However, the need for periodic calibration is a notable consideration for this method. This approach enables precise measurement with low power consumption and can be enhanced with biocompatible coatings to improve sensor accuracy. These biocompatible coatings, such as TPU-based biomembranes, extend the functional lifespan of the probe under the skin. Subcutaneous sensors provide highly accurate data, making them the most preferred method for continuous measurement.

Optical sensors detect glucose molecules through light absorption, reflection, or scattering, enabling non-invasive or minimally invasive measurements. Techniques such as Raman spectroscopy, near-infrared spectroscopy, or fluorescence can be used to analyze glucose in the interstitial fluid beneath the skin. One of the most significant advantages of optical sensors is their ability to function without requiring calibration over long-term use and their durability without the use of enzymes.

Microfluidic sensors analyze interstitial fluid by passing it through microscopic channels. These sensors provide high accuracy with low-volume fluid analysis and allow the simultaneous monitoring of multiple biomarkers (e.g., lactate or ketones). Microfluidic systems offer users a flexible measurement experience due to their portable and modular design. Although still in experimental use, efforts are ongoing to commercialize this technique.

Electromagnetic sensors determine glucose levels by measuring the interaction of glucose molecules with electromagnetic radiation at specific wavelengths. These sensors can be used in minimally invasive or entirely non-invasive applications. Electromagnetic sensing can filter out the effects of other biological factors while measuring glucose levels, offering high accuracy for long-term use. However, stability and medical-grade accuracy have not yet been achieved in these sensors.

Implantable nanomaterial sensors, placed under the skin, utilize advanced nanomaterials such as carbon nanotubes or graphene. Supported by biocompatible coatings, these sensors ensure reliable long-term use and present a low risk of biological reactions. Nanomaterial sensors stand out for their high sensitivity and low energy consumption.

Non-enzymatic electrochemical sensors detect glucose molecules directly through electrochemical oxidation. The non-enzymatic structure enhances the sensor's durability and minimizes the need for calibration. This feature makes it an ideal option for long-term subcutaneous use and positions it as a significant alternative for continuous glucose monitoring devices.

The glucose sensor probe is designed to measure glucose concentrations in interstitial fluid accurately and continuously. This sensor determines glucose levels using electrochemical reactions and transmits the results to an external device via a wireless communication unit. The structural components and working principle of the sensor are detailed below:
The sensor (1) includes a three-layer electrode system composed of platinum, gold, and carbon. Electrical and chemical isolation between the electrodes is provided by a thin polymer layer. This structure allows each layer to function independently and minimizes potential interactions between electrodes. This electrode system, one of the key components of the device, consists of a reference electrode (4), a working electrode (5), and a counter electrode (6). The functions and working principles of each layer are explained in detail below:
First Layer - Reference Electrode (4): The first layer, the reference electrode (4), provides a baseline value for the sensor's operation by measuring the existing voltage levels in the body. This baseline value creates a reference point to understand the ionic balances in the interstitial fluid and the voltage differences between other electrodes. The reference electrode (4) is typically made of biocompatible materials like platinum or silver chloride (Ag/AgCl), which ensure electrochemical stability. By sensitively detecting electrical potential changes in the subcutaneous microenvironment, the reference electrode (4) ensures the accuracy of the device's calibration.

### Integrated Circuit Processing

The voltage value measured by the reference electrode is transmitted to an integrated circuit within the device. The integrated circuit processes this voltage, converting it into a higher voltage output. During processing, the signal is filtered from environmental noise and optimized for transmission to other components of the system. This process is critical for the proper functioning of the second layer, the working electrode. In addition to amplifying the voltage, the integrated circuit provides current control to enhance the system's stability.

Second Layer - Working Electrode (5): The working electrode (5) receives the processed and amplified voltage from the integrated circuit and initiates the electrochemical reactions essential for measuring glucose levels. This layer is coated with biosensor enzymes such as glucose oxidase or glucose dehydrogenase. These enzymes catalyze the oxidation reaction of glucose molecules, generating an electron flow as a result. The electron flow produces an electrical signal proportional to the glucose concentration. The working electrode (5) is typically made of biocompatible materials with high conductivity, such as gold or platinum. The micro-porous structure on the electrode surface increases the reaction surface area, maximizing measurement sensitivity.

Third Layer - Counter Electrode (6): The final layer, the counter electrode (6), generates a lower voltage compared to the working electrode (5). The voltage difference between these two electrodes allows the sensor to measure glucose concentration accurately. The counter electrode (6) balances the current generated by the working electrode, ensuring the stability of the electrochemical cell. It is generally made of a carbon-based structure, which enhances the sensor's performance due to its durability and cost-effective production. Additionally, the electrochemical stability of carbon ensures that the counter electrode functions reliably over long-term use.

### Working Principle

The sensor's measurement cycle begins with the processing of the voltage received from the reference electrode (4) in the integrated circuit. The processed voltage is transmitted to the working electrode (5), initiating electrochemical reactions such as glucose oxidation. During these reactions, the electron flow generated creates a voltage difference between the working and counter electrodes. This voltage difference is analyzed by a microprocessor to determine the glucose level in the interstitial fluid. The measured value is transmitted via a wireless communication unit to a smartphone, smartwatch, or health monitoring device.

This multilayer structure and electrochemical measurement method enable the sensor to perform highly accurate and continuous measurements. The separation of the electrode system by polymer insulators prevents electrical interference between layers and maintains the overall stability of the sensor. Additionally, the biocompatible structure of the sensor (1) probe minimizes the risk of allergic reactions or irritation during long-term subcutaneous use. To achieve this, the glucose sensor probe is initially coated with the enzyme Glucose Oxidase (GOx). This enzyme facilitates a biochemical reaction to detect glucose and produces electrochemical signals. Furthermore, it reacts exclusively with glucose, ensuring high selectivity. Subsequently, the probe is covered with a biomembrane as the final layer. This biomembrane coating minimizes the body's bioreactivity, allows the passage of multiple analytes and water, enhances mechanical durability and flexibility, and prevents clot formation upon contact with blood. These features make the device an ideal solution not only for diabetes management but also for other medical applications requiring glucose monitoring. In one preferred embodiment of the invention, at least a part of the glucose sensor probe system is coated with a bioenzyme and/or biomembrane.

The device includes a microprocessor that processes the electrical signals measured by the glucose sensor probe (tip of the sensor (1)). The microprocessor runs algorithms that analyze raw signals to determine glucose levels. Thanks to the probe's three-layer structure, the device requires less frequent calibration compared to traditional sensors. Additionally, the device is equipped with an automatic calibration mechanism, ensuring accuracy even during extended use. Measurement results are transmitted via a wireless communication module to an external device, such as a smartphone or smartwatch. Low-energy consumption protocols, such as Bluetooth or NFC, are used for wireless communication, enabling users to monitor their glucose levels in real time. Furthermore, the device's algorithms can detect hypoglycemia or hyperglycemia and send early warning alerts.

The microprocessor processes the high-precision data provided by the probe's multilayer structure, accurately calculating glucose levels. The need for manual calibration, common in traditional sensors, is eliminated by the integrated automatic calibration mechanism. This system ensures reliable measurements by maintaining the device's accuracy over long-term use. The microprocessor periodically transmits measurement results to an external mobile device using a Bluetooth infrastructure. This wireless communication system is designed with a low-energy consumption protocol, allowing users to monitor their glucose levels in real time.

The data processed by the microprocessor is collected in a database and periodically shared with a user's mobile device via a low-energy Bluetooth infrastructure, forming an Internet of Things (IoT) system. The shared data is processed within a dedicated user application, visualized as graphs, and stored in a long-term record system. The embedded system within the sensor minimizes the risk of data loss by continuing to store data temporarily in case of a Bluetooth disconnection. This ensures that the device operates continuously to maintain user safety. Additionally, if the sensor detects abnormal glucose levels, it activates the alert system to notify the user immediately.

### Sensor Alert System

The device is equipped with a multilayered alert system designed to notify users of abnormal glucose levels. This system operates in three stages, starting with visual and auditory alerts, followed by physical feedback through vibration if the initial alerts are ignored, and ending with an emergency procedure if no response is received to the physical feedback. These stages are designed to ensure that the user is promptly and effectively informed of potential health risks.

Visual and Auditory Alerts: When glucose levels deviate from the normal range, the system first provides visual and auditory alerts through the mobile application. These alerts are designed to be easily noticeable, allowing the user to take immediate action.

Activation of the Vibration Motor: If the user does not respond to these alerts, the sensor's micro motor is activated, providing a physical alert in the form of vibration. This feature is particularly important in scenarios such as sleep, where visual and auditory alerts may go unnoticed. To address potential issues such as device detachment caused by vibration, advanced adhesive technologies have been employed. Medical-grade adhesives with high resistance to vibration have been specifically selected, ensuring strong adhesion without causing skin irritation or discomfort during prolonged use. These adhesives are optimized for different skin types, allowing the device to adapt to individual needs. In the preferred configurations of the invention, a layered adhesive system has been utilized to enhance performance. The first layer adheres gently to the skin, while the second layer features a flexible structure that absorbs forces generated during vibration. Additionally, micro-porous designs on the adhesive surface improve air and sweat permeability, enhancing both user comfort and the durability of the adhesive connection. In another configuration, a damping layer is integrated between the underside of the device and the adhesive. This layer prevents vibration energy from concentrating at the connection point, reducing stress on the adhesive and ensuring the device remains securely in place. Adhesive technologies used in this invention are optimized for resistance to sweat, skin movement, and environmental factors, ensuring reliable long-term use. Furthermore, the vibration intensity can be adjusted through a user-specific mechanism to minimize the risk of device detachment. This feature allows the vibration strength to be tailored to the user's needs, ensuring effective alerts without compromising the device's position. The device also offers multiple alert modes that enable it to operate at lower vibration levels in synchronization with visual and auditory alerts. Additionally, the invention incorporates an expanded vibration area and damping materials integrated into the device, distributing vibration energy evenly. This design enhances the efficiency of the vibration alerts while maintaining the device's secure attachment and functionality. These features make the device reliable, user-friendly, and highly effective for continuous glucose monitoring.

Emergency Procedure: If the user does not respond to the vibration alert, the device activates its emergency protocol. Within this protocol, pre-defined emergency contacts are called sequentially, and an automated help message is delivered. This message informs the recipients that the user may be experiencing a potential shock, providing critical information to facilitate emergency intervention.

In cases where data transmission fails, such as due to a Bluetooth disconnection or the mobile device running out of battery, the sensor's alert system could face risks. However, the device is equipped with a cloud-based backup module that continues to evaluate data even if transmission is interrupted. If a Bluetooth disconnection occurs, the embedded system within the sensor analyzes the data stored in its backup memory. If an abnormal condition is detected, the vibration motor is activated, ensuring that user safety is maintained even when the connection between the sensor and the mobile device is lost.

Doctor Reporting System: All glucose data collected by the device is comprehensively reported through the user's mobile application. These reports can be shared directly with the user's doctor, with the user's consent. The mobile application visualizes glucose levels in the form of graphs, enabling the doctor to analyze the patient's long-term data more effectively. This system allows the doctor to adjust insulin or other medication dosages with greater precision. Additionally, it helps the doctor create a more tailored diet plan based on the patient's glucose data. This functionality provides a significant advantage for improving health management and optimizing diabetes control for users.

The device uses a long-lasting micro lithium-ion battery as its primary energy source. Alternatively, it can be equipped with energy harvesting technology that collects energy from body movements. This innovative energy management system ensures uninterrupted operation of the device while minimizing the need for battery replacement. All components of the probe and the device are protected by a biocompatible capsule. This capsule prevents the sensor and electronic units from being affected by external conditions, providing a safe environment for long-term subcutaneous use. Additionally, the device is supported by an ergonomic fixation mechanism to secure it to the skin surface. The fixation mechanism consists of a skin-friendly adhesive base and a support element that ensures the probe is positioned at the correct angle. Moreover, the device includes an integrated temperature sensor. This sensor measures body temperature, digitizes the data, and transmits it to a mobile device, offering additional useful information.

By measuring glucose levels from interstitial fluid, the device enables continuous and painless monitoring. The innovative design of the probe provides the precision and accuracy necessary to replace traditional invasive methods. These features not only improve user comfort but also facilitate the early detection of potential risks such as hypoglycemia and hyperglycemia. The invention offers a practical solution not only for the daily management of diabetes patients but also for athletes, pregnant women, and other at-risk groups.

Another alternative is wearable device designs. Lightweight, flexible, and ergonomic, these devices can be easily integrated into users' daily lives. The durability of the device can be further enhanced with waterproof coatings or elastic wristbands.

This innovative design requires less intervention compared to traditional methods, increases user comfort, and allows for continuous glucose monitoring. By combining advanced technology with user-centric design, the device represents a significant advancement in glucose monitoring systems.

Wireless data transmission is another alternative application that enhances the user experience of CGM (Continuous Glucose Monitoring) devices. Protocols such as Bluetooth Low Energy (BLE), Zigbee, or LoRa can be utilized for continuous data transfer. Additionally, by employing data packaging algorithms that minimize energy consumption, the battery life of the device can be extended.

An alternative approach for data transmission and storage systems involves real-time signal processing algorithms. These algorithms effectively perform tasks such as noise filtering and signal accuracy enhancement, enabling more reliable measurement results.

Energy management presents a significant area for improvement in CGM devices. For instance, systems that generate energy from kinetic movement or body heat can reduce the need for batteries. Furthermore, low-power consumption features, such as sleep mode, can contribute to energy efficiency.

Another innovative feature that can be integrated into glucose monitoring devices is the use of predictive and analytical algorithms. Machine learning algorithms can analyze trends in glucose levels, allowing users to anticipate conditions such as hypoglycemia or hyperglycemia before they occur.

The present invention, in a preferred embodiment, incorporates an innovative system integration that enhances the data collection, analysis, and communication capabilities of continuous glucose monitoring (CGM) devices. The device continuously monitors the user's glucose levels via its microprocessor and shares this data in real-time through a dedicated mobile application. Additionally, the integration of the data collected by the device with Turkey's e-Government (e-Devlet) platform offers a solution that facilitates access to healthcare services and streamlines doctor-patient communication.

With e-Government integration, glucose measurements and other biometric data collected by the device can be directly added to the user's health records. This allows the individual's doctor to access these records online, enabling a more comprehensive and efficient monitoring of the patient's health status. Furthermore, the device utilizes an advanced algorithm to analyze the collected data and assess potential health risks. If a potential health issue is detected, the device can automatically notify the doctor, providing critical insights for timely medical intervention.

In emergency scenarios, if the device's algorithm detects a critical glucose level or other signs of an emergency, it can instantly notify the user's doctor and the nearest hospital, facilitating ambulance coordination. This feature elevates the device beyond being merely a monitoring tool, transforming it into a proactive healthcare support system.

The device's data management and emergency response capabilities enhance user comfort while enabling doctors to manage their patients more effectively and prevent potential health crises. By establishing a new standard in personal health tracking, the invention aims to improve individuals' access to healthcare services and support timely medical interventions.

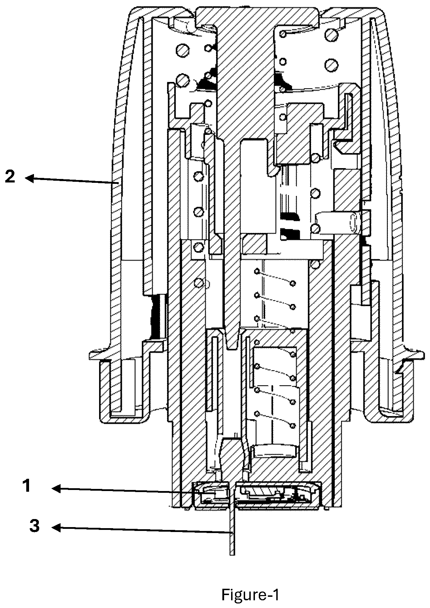

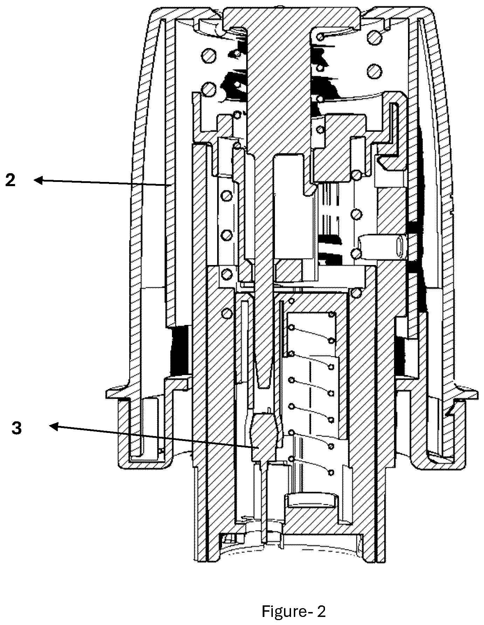

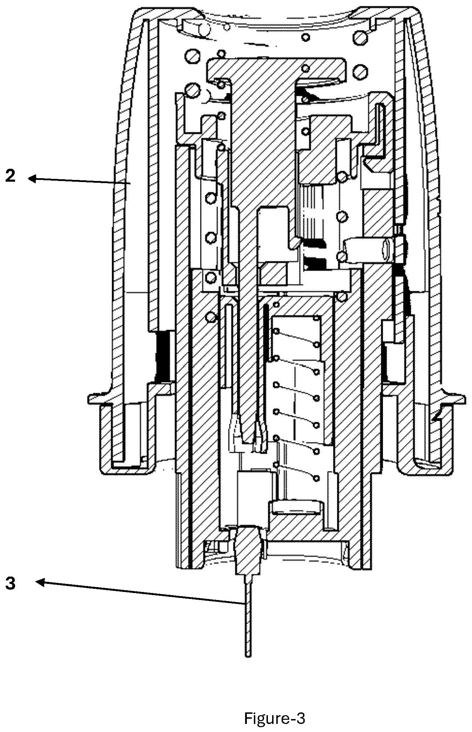

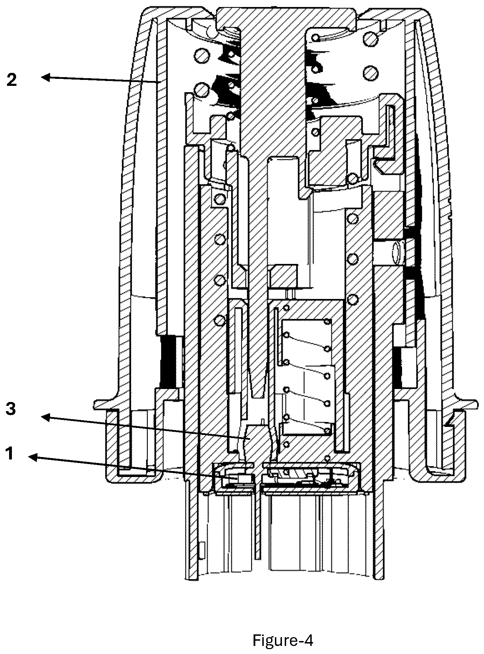

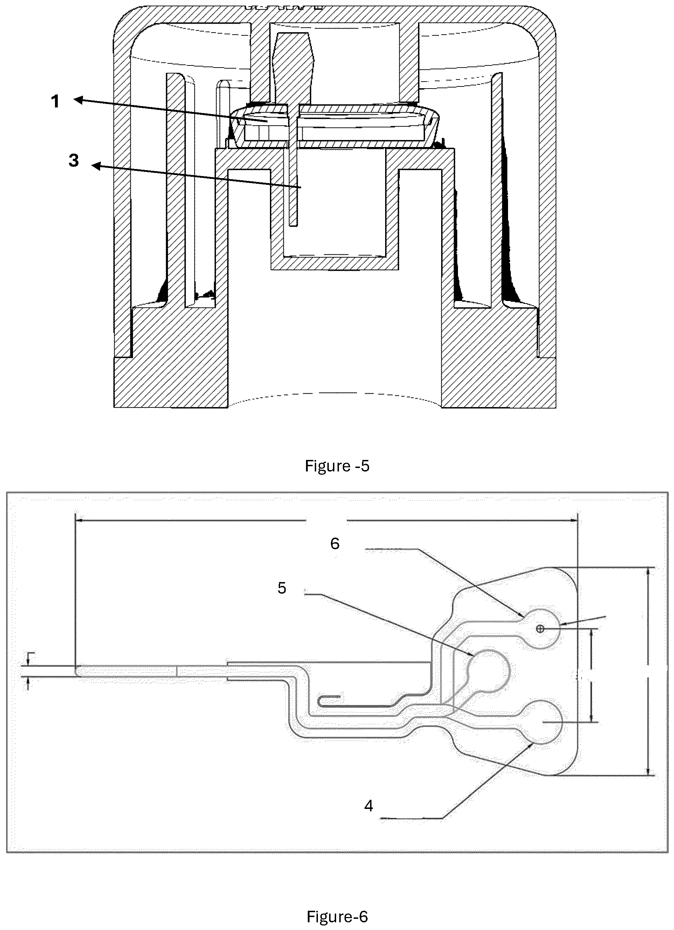

Cross-sectional views of the device are presented in Figures 1 through 5. In Figure 1, the device is shown during the moment of applying pressure on the skin to insert the glucose sensor probe subcutaneously. At this stage, a needle penetrates the skin to position the sensor probe underneath while the sensor module, containing the sensor and communication electronics, is adhered to the skin surface. In Figure 2, the sensor (1) is shown separated from the applicator (2). At this point, the guide needle (3) has fulfilled its function, detaching completely from the sensor and probe and retracting for user safety. Figure 3 demonstrates how the needle, having completed its function and detached from the sensor (1), is hygienically removed from the applicator by pressing the rear trigger mechanism without direct hand contact. This ensures the applicator (2) is ready for reloading with a new sensor and needle. In Figure 4, the trigger button is shown in the upward (non-pressed) position. At this stage, the applicator must first be locked into position, after which a new sensor and needle are loaded into the applicator (2) by pressing them into the loading chamber from above, aligning them with the bed system. The applicator (2) is now ready for the insertion of a new sensor module, as depicted in Figure 4. The sensor module and needle are visible inside the applicator, prepared for use. The next step involves pressing the applicator against the skin (commonly on the triceps area of the arm) to trigger the process and initiate the procedure. Finally, Figure 5 displays the sensor chamber where the sensor (1) and needle are positioned before use. This stage illustrates the device in its pre-use condition, showcasing the readiness of the sensor and needle for application.

The invention subject to the application is a continuous glucose measurement (CGM) device comprising a sensor (1) module comprising a glucose sensor probe system inserted into the subcutaneous interstitial fluid of the user, comprising a three-layer electrode that measures values such as glucose concentration and converts them into an electrical signal, and an electronic unit in which the voltage values received from this system are analysed, a reusable applicator (2) module comprising an automated insertion mechanism configured for accurate insertion of the sensor (1) module it houses into the user's body prior to application, and a sensor insertion structure comprising a sensor insertion structure using a non-touch replaceable guide needle (3) adapted for immediate penetration into the skin by means of a reconfigurable spring-loaded mechanical pushing device, which, when activated by the user pressing on the skin surface, inserts the glucose sensor probe under the skin at a predetermined angle and depth, a microprocessor that processes the measurements obtained by the sensor module (1), a data transmission system that includes a software interface that enables all biometric data of the user obtained to be processed in real time, regularly transmitted or integrated to the user and/or all predetermined devices and units, relevant institutions and remotely accessible digital health platform.

In one preferred embodiment of the invention, the three-layer electrode system comprises a reference electrode (4) which provides a base value for the operation of the sensor by measuring the current voltage level in the body, a working electrode (5) which receives the processed and amplified voltage from the integrated circuit and initiates electrochemical reactions which play a fundamental role in the measurement of glucose levels, and a counter electrode (6) to complete the electrochemical reaction and provide a reference point for accurately measuring the glucose level.

An intelligent health monitoring system is also within the scope of protection of the invention. The health monitoring system comprising a data acquisition unit for receiving data of glucose measurements and/or other health measurements provided by a continuous glucose measurement device according to any embodiment of the present invention and/or at least one different device implanted in the body by the user, a detection unit enabling the user to make an assessment based on past glucose measurements and reference glucose measurements and/or other health measurements, a recommendation unit that allows the user to create a recommendation based on past glucose measurements and social reference measurements of the user's health status, a data transmission unit that includes a software interface that enables all biometric data, assessments and/or recommendations of the user to be processed in real time and regularly transmitted/integrated to the user and/or all predetermined devices and units, relevant institutions and remotely accessible digital health platform and an alarm unit that ensures that warnings and alarms related to this data are transmitted to the relevant devices and systems. Furthermore, the system includes a data storage unit configured to temporarily store data from the devices in the data storage unit in case of disconnection from other devices and to transmit this data to the communication unit when the connection is restored.

Finally, the method of operation of this system is also protected. Three stages are realised in the alarm unit related to the system. These stages are as follows:
in the first stage;
   - The device generates a visual alert (e.g., display indicator or LED light) and an auditory alert (e.g., audible alarm or mobile device notification) if glucose levels exceed a predetermined threshold,
   - These alerts are transmitted to the user via an application via a mobile device,
in the second stage;
   - If the user does not respond to the visual and auditory warnings given in the first stage within a certain period, the device generates a physical warning through a vibration unit,
in the third stage;
   - In case the user does not respond to the vibrating warning in the second stage, an emergency procedure is initiated,
   - Within the scope of this procedure, the device calls or automatically sends a help message to one or more predefined emergency contacts via the mobile device or the intercom module,
   - This message contains information informing the user that the user's glucose level has reached a critical level and urgent intervention is required

The device subject to the invention has been developed to make the sensor placement process safe and user-friendly. The applicator not only ensures that the sensor is positioned at the correct depth and angle but also allows the procedure to be completed quickly and painlessly. Furthermore, both the sensor and applicator components have been designed to ensure durability and suitability for long-term use.

### Explanations of Figures:

**Figure-1** A cross-sectional view of the device of the invention subject to the application during the pressure application.
**Figure 2** A cross-sectional view of the needle (3) of the device of the invention subject to the application after pressure application
**Figure-3** A cross-sectional view of the device of the invention subject to the application, showing the needle (3) being discarded without being touched following the pressure application
**Figure-4** A cross-sectional view of the device of the invention subject to the application ready for reapplication of pressure
**Figure- 5** A cross-sectional view of the device of the invention subject to the application, including the sensor (1) and needle (3) chamber
**Figure- 6** Glucose sensor probe system

### Explanations of Reference Numbers in the Figures:

1. Sensor
2. Applicator
3. Guide needle
4. Reference electrode
5. Working electrode
6. Counter electrode

The concrete examples described here are not intended to limit the scope of the invention. The scope of protection of the invention should be determined according to the following claims, and all technical ideas falling within the equivalents of these claims should also be considered as part of the scope of this invention.

## Claims

1. A continuous glucose measurement (CGM) device, **characterised by** comprising;
- a sensor (1) module comprising a glucose sensor probe system inserted into the subcutaneous interstitial fluid of the user, comprising a three-layer electrode that measures values such as glucose concentration and converts them into an electrical signal, and an electronic unit in which the voltage values received from this system are analysed,
- a reusable applicator (2) module comprising an automated insertion mechanism configured for accurate insertion of the sensor (1) module it houses prior to application, into the user's body, and a sensor insertion structure comprising an insertion structure using a non-touch replaceable guide needle (3) adapted for immediate penetration into the skin by means of a reconfigurable spring-loaded mechanical pushing device, which, when activated by the user pressing on the skin surface, inserts the glucose sensor probe under the skin at a predetermined angle and depth,
- a microprocessor that processes the measurements obtained by the sensor module (1),
- a data transmission system that includes a software interface that enables all biometric data of the user obtained to be processed in real time, regularly transmitted or integrated to the user and/or all predetermined devices and units, relevant institutions and remotely accessible digital health platform.

2. The device according to claim 1, wherein the three-layer electrode system comprises a reference electrode (4) which provides a base value for the operation of the sensor by measuring the current voltage level in the body, a working electrode (5) which receives the processed and amplified voltage from the integrated circuit and initiates electrochemical reactions which play a fundamental role in the measurement of glucose levels, and a counter electrode (6) to complete the electrochemical reaction and provide a reference point for accurately measuring the glucose level.

3. The device according to any one of the preceding claims, wherein the sensor (1) module comprises an adhesive structure which enables the device to be fixed to the skin of the user by means of an adhesive pad in the part where the module (1) is in contact with the skin and; this adhesive structure comprises two or more layers and at least one of these layers is in direct contact with the skin and this layer comprises a skin-friendly adhesive material and at least one other layer comprises a flexible and shock absorbing and/or vibration damping material.

4. The device according to claim 3, wherein the adhesive material comprising the layer of the adhesive structure in direct contact with the skin comprises acrylate/methacrylate copolymer blends, silicone-based adhesives or hydrogel/hydrocolloid-based adhesives or mixtures of two or more thereof, and the material comprising at least one other layer comprises a polyurethane (PU) elastomer or foam layer, a silicone gel layer or a thermoplastic elastomer (TPE) or santoprene-based layer or mixtures of two or more thereof.

5. The device according to any one of the preceding claims, wherein the guide needle (3) has an internal channel structure into which the electrode is inserted.

6. The device according to any one of the preceding claims, wherein it comprises a cloud-based backup module that enables, in the event of a disconnection, the data received from the sensor (1) to be recorded and transmitted to the user and/or to all predetermined devices and units, relevant institutions and a remotely accessible digital health platform and/or to activate the vibration module.

7. The device according to any one of the preceding claims, wherein the insertion mechanism comprises a needle ejection button which activates a spring to allow the guide needle (3) to be withdrawn and discarded untouched after insertion of the sensor (1) has been completed.

8. The device according to any one of the preceding claims, wherein the sensor (1) comprises a waterproof housing, configured to ensure that the operation of the device is not impeded by exposure to moisture, sweat or water on the skin.

9. The device according to any one of the preceding claims, wherein it comprises a built-in temperature sensor and/or that the applicator (2) module comprises a child lock.

10. The device according to any one of the preceding claims, wherein it comprises a vibration module which is activated as a result of data processed in an external device being transferred to the device via a data transmission system.

11. The device according to any one of the preceding claims, wherein the wireless connection is Bluetooth.

12. The device according to any one of the preceding claims, wherein at least a part of the glucose sensor probe system is coated with a bioenzyme and/or biomembrane.

13. A health status monitoring system, **characterised in that** it comprises;
- a data acquisition unit for receiving data of glucose measurements and/or other health measurements provided by a device according to any one of claims 1 to 12 and/or at least one different device implanted in the body by the user,
- a detection unit that allows the user to be assessed according to past glucose measurements and reference glucose measurements and/or other health measurements,
- a recommendation unit that allows the user to create a recommendation based on past glucose measurements and social reference measurements of the user's health status,
- a data transmission unit that includes a software interface that enables all biometric data, assessments and/or recommendations of the user to be processed in real time and regularly transmitted/integrated to the user and/or all predetermined devices and units, relevant institutions and remotely accessible digital health platform,
- an alarm unit that ensures that warnings and alarms related to this data are transmitted to the relevant devices and systems.

14. The system according to claim 13, wherein it comprises a data storage unit configured to temporarily store data from the devices in the data storage unit in the event of disconnection from the other devices and to transmit the data to the communication unit when the connection is restored.

15. A method of operation of a system according to any one of claims 13 or 14, **characterised by** comprising three stages relating to the alarm unit,
In the first stage;
- The device generates a visual alert and an auditory alert if glucose levels go beyond a predetermined threshold,
- These alerts are transmitted to the user via an application via a mobile device,
In the second stage;
- If the user does not respond to the visual and auditory warnings given in the first stage within a certain period, the device generates a physical warning through a vibration unit,
In the third stage;
- In case the user does not respond to the vibrating warning in the second stage, an emergency procedure is initiated, wherein
- Within the scope of this procedure, the device calls or automatically sends a help message to one or more predefined emergency contacts via the mobile device or the intercom module, and
- This message contains information informing the user that their glucose level has reached a critical level and that urgent intervention is required.
